# EUROPÄISCHE PATENTSCHRIFT

(11) **EP 1 980 241 B1**
(45) Veröffentlichungstag und Bekanntmachung des Hinweises auf die Patenterteilung: **14.10.2015**
(21) Anmeldenummer: 07405368.7
(22) Anmeldetag: 24.12.2007
(51) Int. Cl.: A61K 9/00, A61L 15/44, A61L 15/46, A61L 15/40, A61K 8/02

(54) **Feuchttücher zur Intimpflege für Frauen mit Vitex agnus-castus Extrakt**
Wet wipes with vitex agnus castus extract for feminine personal care
Lingettes destinées au soin intime pour femmes contenant de l'extrait de vitex agnus-castus

(30) Priorität: 04.04.2007 CH 5432007
(43) Veröffentlichungstag der Anmeldung: 15.10.2008
(73) Patentinhaber: Mibelle AG, 5033 Buchs (CH)
(72) Erfinder: Zülli, Fred, 5024 Küttigen (CH); Suter, Franz, 5312 Döttingen (CH); Schürch, Cornelia, 5600 Lenzburg (CH); Schmid, Daniel, 5200 Brugg (CH)
(74) Vertreter: Rottmann, Maximilian

(56) Entgegenhaltungen:
- US-A1- 2003 120 224
- US-A1- 2003 144 639
- US-A1- 2003 170 325
- US-A1- 2005 186 255

## Beschreibung

Die vorliegende Erfindung betrifft Feuchttücher, sog."Wet Wipes", zur Intimpflege für Frauen unter Verwendung einer flüssigen Formulierung mit beruhigenden und lindernden Eigenschaften. Im Besonderen enthält die genannte Flüssigformulierung einen Extrakt aus den trockenen Früchten von Mönchspfeffer (*Vitex agnus-castus),* gegebenenfalls in Kombination mit einem Extrakt aus einem Kaktus der Gattung *Opuntia* und/oder CM-Glucan. Diese Feuchttücher werden zur Reinigung der äusseren Schleimhäute des Intimbereiches eingesetzt.

Bereits in jungen Jahren ist eine gute Intimpflege wichtig, später erst recht. Der Mensch wird älter und damit seine Haut und Schleimhäute. Sie werden dünner, trockener und empfindlicher. Dabei spielt der Östrogenrückgang in den Wechseljahren eine grosse Rolle. Die Intimhaut ist ständigen Strapazen durch Seife, Sauna, Sprudelbäder, Slipeinlagen, Schwitzen, Unterwäsche und auch Stress ausgesetzt. Die Intimreinigung und -pflege ist von immenser Bedeutung für das Wohlbefinden und die Gesundheit der Frauen.

Es ist allgemein bekannt, dass eine gesunde Intimschleimhaut stark von einer intakten Vaginalmikroflora abhängt. Um die Erhaltung der Mikroflora zu gewährleisten, ist ein leicht saures Milieu mit einem pH-Wert von 3,5 bis 4,5 notwendig.

Viele Intimpflegeprodukte enthalten Milchsäure oder ein Citrat um den entsprechend tiefen pH-Wert für eine gesunde Mikroflora zu gewährleisten. Des Weiteren werden besonders milde Seifen ohne Parfum oder Konservierungsmittel oder anderen potentiell allergisierenden Zusatzmitteln verwendet, die oft auch antibakterielle oder entzündungshemmende Substanzen, wie zum Beispiel Kamille, Salbei oder Thymian, enthalten.

Derartige Flüssigformulierungen werden üblicherweise in Form von Waschlotionen oder Duschbädern eingesetzt, oder sie werden auf verschiedenartige Gewebe aufgetragen, wie zum Beispiel Baumwolle oder synthetische Materialien, zur Verwendung als Hygiesynthetische Materialien, zur Verwendung als Hygienetücher. Die wichtigsten Patentschriften auf diesem Gebiet sind: US 4 788 060, MX PA04003088 A, WO 03/051227 A2, US 2005/186255 A1, CN 1557288 A.

Aus der Veröffentlichung US 2003/0120224 A1 sind Produkte für die Verabreichung therapeutischer Wirkstoffe an das Scheidenepithel zur systemischen und topischen Behandlung bekannt. Entsprechend ihrem Zweck verbleiben die eingesetzten Produkte längere Zeit in Berührung mit dem Scheidengebiet, wo sie wirken sollen. Als Wirkstoff wird u.a. auch Extrakte aus Mönchspfeffer (*Vitex agnus-castus*) erwähnt. Die US 2003/0144639 A1 beschreibt einen Tampon, mit dem ein therapeutischer Wirkstoff verabreicht wird, wobei der Tampon an seinem distalen Ende eine Dosierungsform, die den therapeutischen Wirkstoff enthält, aufweist, die an dem Tampon fixiert ist, und die Dosierungsform durch teilweises Schmelzen einer Befestigungsschicht der Dosierungsform an dem Körper fixiert wird. Die Dosierungsform enthält eine Mehrzahl von Schichten, wobei eine Schicht die Befestigungsschicht ist und in einer weiteren Schicht der Wirkstoff enthalten ist. Unter einer Vielzahl von aktiven Wirkstoffen, sowohl synthetischer als auch pflanzlicher Natur wird Mönchspfeffer genannt.

Mönchspfeffer wird im Volksmund auch "Keuschlamm" genannt. Der Name zeigt die Wirkung, die dieser Pflanze im Altertum zugesprochen wurde. In Klöstern wurden die gemahlenen Samen als Gewürzpulver in hohen Dosen gebraucht, um den Geschlechtstrieb abzuschwächen. Bei geringer Dosierung bewirkt er das Gegenteil.

Heutzutage wird der Mönchspfeffer vor allem zur Linderung des prämenstruellen Syndroms (PMS) und gegen Zyklusstörungen eingesetzt. Die Wirkung ist eine Folge der Hemmung der Prolactinsekretion. Es kommt zu einer Senkung des während des PMS oft pathologisch erhöhten Prolactinspiegels und damit zu einer Normalisierung gestört verlaufender Menstruationszyklen. Hyperprolactinemia führt zu einer unzureichenden Progesteron-Bildung während der Luteinisierung nach dem Eisprung und zu Symptomen wie dem prämenstruellen Syndrom, Zyklusunregelmässigkeiten oder Spannungsgefühlen in den Brüsten (Mastodynie). Pharmakologisch ist die dopaminerge Wirkung von Mönchspfeffer schon lange bekannt. Bestandteile des Extraktes, wahrscheinlich lipophile Diterpene, binden an den Dopamin-D2-Rezeptor an. Mönchspfeffer senkt den Prolactin-Wert, wahrscheinlich durch seine dopaminerge Wirkung (Dopamin-D2-Rezeptor hat eine hemmende Wirkung auf die Prolactin-Freisetzung). Dadurch normalisieren sich die Progesteron-Werte. Die Wirksamkeit von Mönchspfeffer beim PMS kann ebenfalls über die dopaminerge Aktivität zufriedenstellend begründet werden (Hemmung der Prolactinsekretion und positiver emotionaler Effekt). Zusätzlich wurde für verschiedene Mönchspfeffer Fraktionen eine Aktivität an Opioid-Rezeptoren gefunden. Dabei wurden an den µ- und κ-al., Phytomedicine Vol 7 (5), Seiten 373 bis 381, 2000). Im Gegensatz zum D2-Rezeptor, wo nur die Hexan-Unterfraktion eines Methanolextraktes das Binden von Radioliganden verhindern konnte, haben bei den Opioid-Rezeptoren verschieden polare Unterfraktionen eines Methanolextraktes einen hemmenden Effekt auf das Binden von Radioliganden. Diese Aktivität weiter zu verfolgen ist insofern von Interesse, als bei Frauen, die am PMS leiden, beobachtet werden konnte, dass der Abfall des Östrogens und des Progesterons in der spätlutealen Phase mit einer übermässig starken Konzentrationsabnahme des zentralen Endorphins verbunden ist. Eine Beeinflussung der β-Endorphin Konzentration durch Mönchspfeffer wurde auch beobachtet. Der Extrakt führte zu einer signifikanten Erhöhung der β-Endorphin Konzentration im Serum weiblicher Ratten. Es ist anzunehmen, dass der Extrakt dank seiner dopaminagonistischen Wirkungsweise die Prolactinsekretion hemmt und damit durch eine Regulation der Sexualhormone die β-Endorphin Konzentration beeinflusst.

Aufgabe der Erfindung war nun die Schaffung von Feuchttüchem, sog. "Wet Wipes", zur Intimpflege für Frauen unter Verwendung einer flüssigen Formulierung, welche einen Extrakt aus den trockenen Früchten von Mönchspfeffer *(Vitex agnus-castus)* enthält. Diese Feuchttücher werden zur Reinigung der äusseren Schleimhäute des Intimbereiches eingesetzt und nach ihrem kurzen Einsatz zu Reinigungungszwecken fortgeworfen.

Die Verwendung dieses speziellen Mönchspfeffer-Extraktes in einer Formulierung für Intimpflegetücher ist neu und bewirkt, dass Irritationen gelindert werden und dass der Intimbereich befeuchtet und erfrischt wird.

Dass der erfindungsgemässe Wirkstoff überhaupt und im Besonderen in der kurzen Einwirkzeit diese Wirkungen entfalten kann, war für den Fachmann in hohem Masse überraschend. Auf Grund der Lehre der erwähnten Veröffentlichung US 2003/0120224 A1 musste er voraussetzen, dass nur länger dauernde Einwirkungsdauern irgendwelche Wirkungen erzeugen.

Im Einzelnen bewirken die erfindungsgemässen Feuchttücher zur Intimpflege für Frauen folgendes:
- Regulation von Menstruationsproblemen im Genitalbereich;
- Befeuchtung der Vaginalschleimhaut besteht;
- er Regulation von Hautproblemen, die durch hormonelle Schwankungen verursacht werden;
- Beruhigung von Hautirritationen;
- Linderung von Juckreiz;
- Stimulierung des Immunsystems der Vaginalschleimhaut;
- Vorbeugung gegen Infektion mit pathogenen Keimen; sowie
- Kombination der genannten Wirkungen.

Besonders gute Ergebnisse werden erzielt, wenn man die erfindungsgemässen Mönchspfeffer-Extrakte mit Extrakten aus einem Kaktus der Gattung *Opuntia,* insbesondere aus *Opuntia streptacantha stem* oder *Opuntia ficus-indica stem,* und/oder CM-Glucan kombiniert. Dabei zeigte es sich, dass die Wirkungen dieser Kombinationen über die Summe der Einzelwirkungen weit hinaus gehen.

Zur Gattung *Opuntia* gehören zwei- bis dreihundert Kaktusarten, die weltweit in ariden und semiariden Zonen beheimatet sind. Kommerziell wird *Opuntia* in Spanien, Kalifornien, Mexiko, Argentinien und Chile angebaut. Gebraucht werden vor allem die Früchte aber auch andere Kaktusteile werden als Gemüse konsumiert oder finden Verwendung als pflanzliches Arzneimittel oder als Zusatz in Kosmetikprodukten. Die Blätter des Kaktus, die eigentlich den Stamm bilden, werden zu einem Pulver verarbeitet, das reich an Mineralien, Pektin und Flavonoiden ist. Als charakteristische Substanz für *Opuntia* gilt die Piscidsäure. Es ist eine Verbindung von Zimtsäure und Weinsäure. Durch die zwei Carboxylgruppen wirkt Piscidsäure als Chelator und kann so zum Beispiel freie Eisenionen binden und damit unschädlich machen. Freie Eisenionen sind toxisch, weil sie in der Fentonreaktion zur Bildung des extrem reaktiven Hydroxylradikals führen. Das Pulver des Nopal-Kaktus wird hauptsächlich als Nahrungsergänzungsmittel zur Gewichtsabnahme und gegen Diabetes eingenommen. Für die Gewichtskontrolle scheint der hohe Pektingehalt wichtig zu sein. Die anti-diabetes Wirkung wird wahrscheinlich durch Saponine erzeugt. Es gibt mehrere wissenschaftliche Publikationen, die eine schmerzlindernde und eine entzündungshemmende Wirkung von Opuntia-Kaktusextrakt zeigen.

Der Einsatz von einem *Opuntia*-Kaktusextrakt in kosmetischen Mitteln ist bereits bekannt. Der Opuntia-Wirkstoff wird dabei hauptsächlich als Feuchtigkeitsmittel ausgelobt. Die Verwendung eines *Opuntia*-Kaktusextraktes in einer Formulierung für Intimpflegetücher ist hingegen neu und wird hier erstmals vorgeschlagen. Neu ist auch der Einsatz eines *Opuntia*-Kaktusextraktes in Hautpflegemitteln zur Linderung von unangenehmen Hautirritationen und Juckreiz.

CM-Glucan ist die Bezeichnung für ein wasserlösliches β-1→3-verknüpftes Glucan, das aus Bäckerhefe isoliert wurde. CM steht für carboxy-methyliert, ein chemisches Verfahren um das ansonsten unlösliche Glucan in eine wasserlösliche Form überzuführen. Der Einsatz von CM-Glucan als Wirkstoff in kosmetischen Mitteln ist bekannt. Die Mittel stärken das Immunsystem der Haut, wirken der Faltenbildung entgegen und können auch gegen Schuppenflechte und Psoriasis eingesetzt werden. Ein anderes bekanntes Einsatzgebiet ist ihre Verwendung in Sonnenschutzmitteln. Der hier erstmals vorgeschlagene Einsatz in einer Formulierung für Intimpflegetücher ist dagegen neu.

Alle im Folgenden genannten Zahlen sind Gewichtsprozent. Die Bezeichnung der Inhaltsstoffe entspricht der INCI-Nomenklatur (International Cosmetics Ingredients), wie sie zur Kennzeichnung kosmetischer Wirk- und Hilfsstoffe bestimmt ist.

### Beispiele

### 1 Intim-Feuttücher mit einem Extrakt aus Mönchspfeffer (nicht erfindungsgemäss)

| **Phase** | **Ingredients** | **INCI** | % w/w |
|---|---|---|---|
| **W1** | Wasser | Aqua | ad 100 |
| | Natriumbenzoat | Sodium Benzoate | 0,3 |
| | Milchsäure 80% | Lactic Acid | 0,11 |
| **W2** | Softigen 767 | PEG-6 Caprylic/Capric Glycerides | 1,0 |
| | Glycerin | Glycerin | 0,2 |
| **P** | Cremophor CO 60 | PEG-60 Hydrogenated Castor Oil | 1,5 |
| | Parabens in | Parabens in Phenoxyethanol | 0,8 |
| | Phenoxyethanol | | |
| | Polysorbate 20 | Polysorbate 20 | 0,3 |
| | Deolite | Dimethyl Phenylpropanol | 0,2 |
| | Sensiva SC-50 | Ethylhexylglycerin | 0,2 |
| | D-Panthenol 75% | Panthenol | 0,4 |
| | Parfum | Parfum | 0,2 |
| **A** | Mönchspfeffer-Extrakt | *Vitex agnus-castus-Extract* (und) Alcohol (und) Glycerin (und) Aqua/ Water | 1,0 |

### Verfahren:

- Natriumbenzoat (W1) im Wasser (W1) lösen
- Milchsäure (W1) zugeben, rühren bis alles gelöst ist
- W2 einzeln zugeben, einrühren
- P mischen, zu W1+W2 geben
- A nacheinander dazugeben
- Mischen, bis alle Komponenten homogen gelöst sind.

### 2 Intim-Feuchttücher mit Extrakten aus Mönchspfeffer und Opuntia

| **Phase** | **Inhaltsstoffe** | **INCI** | **% w/w** |
|---|---|---|---|
| **W1** | Wasser | Aqua | ad 100 |
| | Natriumbenzoat | Sodium Benzoate | 0,3 |
| | Milchsäure 80% | Lactic Acid | 0,11 |
| | | | |
| **W2** | Cetiol HE | PEG-7 Glyceryl Cocoate | 0,6 |
| | Glycerin | Glycerin | 0,2 |
| | | | |
| **P** | Cremophor CO 60 | PEG-60 Hydrogenated Castor Oil | 1,5 |
| | Parabens in | Parabens in Phenoxyethanol | 0,8 |
| | Polysorbate 20 | Polysorbate 20 | 0,3 |
| | Deolite | Dimethyl Phenylpropanol | 0,2 |
| | Sensiva SC-50 | Ethylhexylglycerin | 0,2 |
| | α-Bisabolol | Bisabolol | 0,1 |
| | Parfum | Parfum | 0,2 |
| | | | |
| **A** | Mönchspfeffer-Extrakt | *Vitex agnus-castus-*Extract (und) Alcohol (und) Glycerin (und) Aqua/ Water | 1,0 |
| | | | |
| | *Opuntia*-Extrakt | *Opuntia streptacantha stem*-Extract (und) Glycerin (und) Phenoxyethanol (und) Aqua/Water | 1,0 |

### Verfahren:

- Natriumbenzoat (W1) im Wasser (W1) lösen
- Milchsäure (W1) zugeben, rühren bis alles gelöst ist
- W2 einzeln zugeben, einrühren
- P mischen, zu W1+W2 geben,
- A nacheinander dazugeben
- Mischen, bis alle Komponenten homogen gelöst sind

### 3 Intim-Feuchttücher mit einem Extrakt aus Mönchspfeffer und mit CM-Glucan (nicht erfindungsgemäss)

| **Phase** | **Ingredients** | **INCI** | **% w/w** |
|---|---|---|---|
| **W1** | Wasser | Aqua | ad 100 |
| | Natriumbenzoat | Sodium Benzoate | 0,3 |
| | Milchsäure 80% | Lactic Acid | 0,11 |
| **W2** | Cetiol HE | PEG-7 Glyceryl Cocoate | 0,6 |
| | Glycerin | Glycerin | 0,2 |
| **P** | Cremophor CO 40 | PEG-40 Hydrogenated Castor Oil | 1,3 |
| | Parabens in Phenoxyethanol | Parabens in Phenoxyethanol | 0,8 |
| | Polysorbate 20 | Polysorbate 20 | 0,3 |
| | Propylenglycol USP | Propylene Glycol | 2,0 |
| | Sensiva SC-50 | Ethylhexylglycerin | 0,2 |
| | α-Bisabolol | Bisabolol | 0,1 |
| | Parfum | Parfum | 0,2 |
| **A** | Mönchspfeffer-Extrakt | *Vitex agnus-castus*-Extract (und) Alcohol (und) Glycerin (und) Aqua/ Water | 1,0 |
| | CM-Glucan P | Sodium Carboxymethyl Betaglucan (und) Phenoxyethanol (und) Aqua/ Water | 1,0 |

### Verfahren:

- Natriumbenzoat (W1) im Wasser (W1) lösen
- Milchsäure (W1) zugeben, rühren bis alles gelöst ist
- W2 einzeln zugeben, einrühren
- P mischen, zu W1+W2 geben,
- A nacheinander dazugeben
- Mischen, bis alle Komponenten homogen gelöst sind

### 4 Intim-Feuchttücher mit Extrakten aus Mönchspfeffer und Opuntia und mit CM-Glucan

| **Phase** | **Ingredients** | **INCI** | **% w/w** |
|---|---|---|---|
| **W1** | Wasser | Aqua | ad 100 |
| | Natriumbenzoat | Sodium Benzoate | 0,3 |
| | Milchsäure 80% | Lactic Acid | 0,11 |
| | | | |
| **W2** | Softigen 767 | PEG-6 Caprylic/Capric Glycerides | 1,0 |
| | Glycerin | Glycerin | 0,2 |
| | | | |
| **P** | Cremophor CO 60 | PEG-60 Hydrogenated Castor Oil | 1,5 |
| | Parabens in Phenoxyethanol | Parabens in Phenoxyethanol | 0,8 |
| | Polysorbate 20 | Polysorbate 20 | 0,3 |
| | Deolite | Dimethyl Phenylpropanol | 0,2 |
| | Sensiva SC-50 | Ethylhexylglycerin | 0,2 |
| | D-Panthenol 75% | Panthenol | 0,4 |
| | Parfum | Parfum | 0,2 |
| | | | |
| **A** | Femitim | *Vitex agnus-castus*-Extract (und) Alcohol (und) Glycerin (und) Aqua/ Water | 1,0 |
| | | | |
| | CM-Glucan P | Sodium Carboxymethyl Betaglucan (und) Phenoxyethanol (und) Aqua/ Water | 1,0 |
| | | | |
| | *Opuntia*-Extrakt | *Opuntia streptacantha stem*-Extract (und) Glycerin (und) Phenoxyethanol (und) Aqua/Water | 1,0 |

### Verfahren:

- Natriumbenzoat (W1) im Wasser(W1) lösen
- Milchsäure (W1) zugeben, rühren bis alles gelöst ist
- W2 einzeln zugeben, einrühren
- P mischen, zu W1+W2 geben,
- A nacheinander dazugeben
- Mischen, bis alle Komponenten homogen gelöst sind

### 5 Intim-Feuchttücher mit Extrakten aus Mönchspfeffer und Opuntia

| **Phase** | **Ingredients** | **INCI** | **% w/w** |
|---|---|---|---|
| **W1** | Wasser | Aqua | ad 100 |
| | Natriumbenzoat | Sodium Benzoate | 0,3 |
| | Milchsäure 80% | Lactic Acid | 0,2 |
| | | | |
| **W2** | Cetiol HE | PEG-7 Glyceryl Cocoate | 0,8 |
| | Glycerin | Glycerin | 0,2 |
| | | | |
| **P** | Cremophor CO 60 | PEG-60 Hydrogenated Castor Oil | 1,0 |
| | Parabens in Phenoxyethanol | Parabens in Phenoxyethanol | 0,8 |
| | Polysorbate 20 | Polysorbate 20 | 0,3 |
| | Deolite | Dimethyl Phenylpropanol | 0,2 |
| | Sensiva SC-50 | Ethylhexylglycerin | 0,2 |
| | D-Panthenol 75% | Panthenol | 0,4 |
| | Parfum | Parfum | 0,2 |
| | | | |
| **A** | Mönchspfeffer-Extrakt | *Vitex agnus-castus*-Extract (und) Alcohol (und) Glycerin (und) Aqua/ Water | 1,0 |
| | | | |
| | *Opuntia*-Extrakt | *Opuntia ficus*-indica *stem*-Extract (und) Glycerin (und) Phenoxyethanol (und) Aqua/Water | 1,0 |

### Verfahren:

- Natriumbenzoat (W1) im Wasser (W1) lösen
- Milchsäure (W1) zugeben, rühren bis alles gelöst ist
- W2 einzeln zugeben, einrühren
- P mischen, zu W1+W2 geben,
- A nacheinander dazugeben
- Mischen, bis alle Komponenten homogen gelöst sind

### 6 Personenbefragung bezüglich Wirksamkeit der Feuchttücher zur Intimpflege

Die Feuchttücher wurden von 20 Frauen im Alter von 23 bis 55 Jahren getestet. Während 4 Wochen wurden die Feuchttücher mindestens 3-mal wöchentlich im Intimbereich angewendet. Vor Beginn der Studie wurden die Testpersonen durch einen Gynäkologen über den richtigen Gebrauch der Feuchttücher informiert. Nach der Studie wurden die Testpersonen im Beisein des Gynäkologen über die Wirksamkeit der Feuchttücher befragt.

### Auswertung des Fragebogens:

| | Antworten total | Antworten positiv (zufrieden oder sehr zufrieden | |
|---|---|---|---|
| | | Zahl | % |
| Wie finden Sie das Gefühl nach dem Gebrauch der Feuchttücher? Wie finden Sie die Wirkung? | | | |
| Gefühl nach Gebrauch | 20 | 17 | 85 |
| Erfrischend | 20 | 16 | 80 |
| Lindert Irritationen | 20 | 14 | 70 |
| Befeuchtung | 20 | 14 | 70 |
| Fühle mich sicherer | 20 | 15 | 75 |
| Fühle mich frischer | 20 | 17 | 85 |
| Fühle mich gepflegt | 20 | 16 | 80 |
| Allgemeines Wohlgefühl | 20 | 17 | 85 |

## Patentansprüche

1. Feuchttuch zur Intimpflege für Frauen, **gekennzeichnet durch** eine Flüssigformulierung mit einem wirksamen Gehalt eines Extraktes aus Früchten von Mönchspfeffer *(Vitex agnus-castus*) und einen wirksamen Gehalt eines Extraktes aus einem Kaktus der Gattung *Opuntia.*

2. Feuchttuch nach Anspruch 1, **dadurch gekennzeichnet, dass** der *Opuntia-*Extrakt ein Extrakt aus *Opuntia streptacantha stem* ist.

3. Feuchttuch nach Anspruch 1, **dadurch gekennzeichnet, dass** der *Opuntia-*Extrakt ein Extrakt aus *Opuntia-ficus-indiaca stem* ist.

4. Feuchttuch nach einem der Ansprüche 1 bis 3, **dadurch gekennzeichnet, dass** die Flüssigformulierung ausserdem einen wirksamen Gehalt an CM-Glucan enthält.

## Claims

1. A wet wipe for feminine intimate care, **characterized by** a liquid formulation comprising an effective content of monk's pepper fruit extract (vitex agnus-castus) and an effective content of a cactus extract of the opuntia genus.

2. The wet wipe as set forth in claim 1, **characterized in that** said opuntia extract is an extract of opuntia streptacantha stem.

3. The wet wipe as set forth in claim 1, **characterized in that** said opuntia extract is an extract of opuntia ficus indiace stem.

4. The wet wipe as set forth in any of the claims 1 to 3, **characterized in that** said liquid formulation contains in addition an effective content of CM-Glucan.

## Revendications

1. Lingette destinée au soin intime pour femmes, **caractérisée par** une formulation liquide avec une quantité active d'un extrait de fruits de gattilier *(vitex agnus-castus)* et une quantité active d'un extrait d'un cactus de l'espèce *opuntia.*

2. Lingette selon la revendication 1, **caractérisée en ce que** l'extrait *d'opuntia* est un extrait de tige *d'opuntia streptacantha.*

3. Lingette selon la revendication 1, **caractérisée en ce que** l'extrait *d'opuntia* est un extrait de tige *d' opuntia ficus indica.*

4. Lingette selon l'une des revendications 1 à 3, **caractérisée en ce que** la formulation liquide contient en outre une quantité active de CM glucane.
